# EUROPEAN PATENT SPECIFICATION

(11) **EP 1 938 768 B1**
(45) Date of publication and mention of the grant of the patent: **19.05.2010**
(21) Application number: 06769528.8
(22) Date of filing: 24.01.2006
(51) Int. Cl.: A61B 19/00, F21V 14/00, F21S 8/00, F21V 23/04

(54) **SURGICAL LIGHT PROVIDED WITH A LIGHT EMISSION CONTROL**
CHIRURGIE-LICHT MIT LICHTEMISSIONSSTEUERUNG
APPAREIL D'ÉCLAIRAGE CHIRURGICAL MUNI D'UNE COMMANDE DE RAYONNEMENT LUMINEUX

(43) Date of publication of application: 02.07.2008
(73) Proprietor: Zakrytoe Aktsionernoe Obschestvo Zavod Ema, Ekaterinburg 620028 (RU)
(72) Inventor: KALETIN, Andrei Aleksandrovich, Ekaterinburg, 620131 (RU); ROSICHINA, Olga Michailovna, Ekaterinburg, 620075 (RU); TSEPELEV, Dmitry Vitalievich, Ekaterinburg, 620102 (RU); OSIPOV, Nikolai Aleksandrovich, Moskovskaya obl., 142432 (RU); VERSHININ, Nikolai Fedorovich, Moskovskaya obl., 142432 (RU)
(74) Representative: Sloboshanin, Sergej
(86) International application number: PCT/RU2006/000021
(87) International publication number: WO 2007/086770

(56) References cited:
- EP-A- 1 568 934
- EP-A- 1 568 935
- EP-A- 1 568 936
- EP-A1- 1 568 935
- JP-A- 2004 200 102
- RU-C1- 2 158 876
- RU-C2- 2 227 245
- RU-C2- 2 235 942
- RU-C2- 2 244 870
- US-A1- 2004 129 860

## Description

According to the present invention, the subject is achieved by a surgery lamp with light emission control defined in the preamble of claim 1.

EP 1 568 936 A1 discloses a lighting unit consisting of a number of modules having a hexagonal shape that fit together to form a dish-shaped unit that focuses light onto a central region. Each module has a number of light emitting, diodes LED as lighting elements of different colors, each with a lens that is coupled to a regulator to control dimming. The optical axis of each module is directed into a focal point of a spherical surface.

EP 1 568 934 A1 discloses an operating lamp which includes a lamp body for receiving a light source, a first light source housed in the lamp body and adapted for illuminating an illumination area of an operating area, and a second light source housed in the lamp body and adapted for illuminating a central portion of the illumination area. The brightness of the second light source is controllable independently of the brightness of the first light source to enhance the brightness of the central portion of the illumination area.

Since the time when light diodes entered everyday practice, illumination devices using lamps consisting of light diodes have enjoyed particular predominance over existing fluorescent lamps, incandescent lamps etc., which is due to their long life span and low energy consumption.

Illumination appliances with light diodes used as projectors or traffic lights are described in detail in Russian Federation Patents like 2123633, 2151473, 2153623, 2202731, 2227245, 2244870, 2248499 and others. They are designed for illuminating faraway objects, for use in systems of all-round surveillance and illuminating appliances where no maximum level of illumination within the operational area and no maintaining of a fixed diameter of the light field are required.

However, to the present time light diodes are not widely applied in surgery lamps, which need to achieve a shadowless effect and a high effectivity of the light due to a high light intensity and an equal distribution of the light beam within the operational area. The light beam of surgery lamps is created by an addition of the light beams of separate light optical modules, which is carried out at a distance of 0.7 m - 1.4 m from the housing. Within the operational area the maximum level of illumination is created, and a constant diameter of the light field is maintained. Moreover there must be a possibility to control the diameter of the light field. To this end, surgery lamps are equipped with devices for controlling the light field, which generally are rotatable mechanical means.

A surgery lamp is known having a mechanism for controlling the dimensions of the light field in the form of a rotatable mirror which reflects the light ray from the light source (Lamp of the BH-500 trade mark of the company FAMED (Poland)).

The mechanism of the known lamp for controlling the dimensions of the light field is not sufficiently effective due to the low synchronisation of the rays, the usage of a superfluous reflecting surface, and it also has large dimensions.

A surgery lamp is known in which the regulation of the illumination system is implemented by displacing the lamp with respect to the reflector (surgery lamp SP-6; No. 66-00-00PS).

The known lamp can regulate only the size of a light spot of a small depth, and it also has large dimensions.

Also, a surgery lamp with light emission control is known, comprising light optical elements in the form of halogen lamps mounted in the housing, and an apparatus for controlling the light field, comprising a bearing rod which is arranged on the main axis of the illumination system, and in the upper part of which a detachable ball bearing is arranged with six degrees of freedom, means for rotating the light optical elements in the form of a multiple-thread cam arranged below the ball bearing and interacting with vertical guides of the holders of the light optical elements, which interact with adjusting pins (Russian Federation Patent 2149309, International Class F21V 14/02, F21V21/29, F21W131:205 published May 20^{th}, 2000).

In the known technical solution the control of the light emission is implemented by mechanical means causing rotation of the light optical elements which have a complicated construction, large dimensions, and the reliability of which decreases with the wear of the mechanical units.

The most pertinent prior art to the claimed apparatus in terms of technical essence is a surgery lamp comprising light optical elements mounted on the housing and connected to one another by a hinge, and an apparatus for controlling the light field by mechanical means (European Patent Application 1568935, International Class F21S 8/00, F21S 2/00, published August 31^{st}, 2005).

The known lamp has a high effectivity of the light. However, the control of the light emission of the known lamp is implemented by mechanical means causing rotation of the light optical elements with respect to each other, the reliability of which decreases with the wear of the mechanical units.

### Disclosure of the invention

The objective of the claimed invention is an increase of the reliability of surgery lamps while maintaining a high effectivity of the light, and it is achieved as follows:

In a surgery lamp with light emission control, comprising light optical elements mounted on the lamp housing and an apparatus for controlling the light field, in accordance with the claimed technical solution the light optical elements have the form of basic light diodes distributed over the surface of the lamp housing, and of additional light diodes located between the basic diodes and having a spatial orientation differing from the spatial orientation of the basic light diodes, wherein the basic and the additional light diodes are gathered in groups,
wherein the groups of basic light diodes and the groups of additional light diodes are connected to each other and to the apparatus for controlling the light field,
wherein the groups of basic and additional light diodes have different spatial orientation allowing to carry out an electronic control of the diameter of the light field, causing thereby a redistribution of the light beam within the light field.

The basic light diodes are mounted on the lamp housing, their optical axes intersecting the axis of the lamp in the centre of the light spot.

The additional light diodes are mounted on the housing, their optical axes forming a concentric light spot in the shape of a ring around the basic spot.

Moreover the apparatus for controlling the light field comprises a control unit to which the groups of basic and additional light diodes are connected by regulators, each of which is connected to a feed unit.

The arrangement of basic light diodes distributed on the lamp housing and the additional light diodes between the basic light diodes, as well as the gathering of the light diodes in groups and the different spatial orientation of the groups of basic and additional light diodes allows to carry out an electronic control of the diameter of the light field, which increases the reliability of the lamp.

The implementation of the apparatus for controlling the light field consisting of a control unit to which groups of basic and additional light diodes are connected by means of regulators, each of which is connected to a feed unit, allows to carry out a correction of the balance of currents running through the groups of basic and additional light diodes, causing a redistribution of the light beam within the light field, i.e. it allows to carry out an electronic control of the light field, increasing the operational reliability of the lamp.

The essential features which distinguish the claimed apparatus from the most pertinent prior art allow to consider it a novelty.

No technical solutions are disclosed in the prior art which correspond to the characterising features of the claimed subject matter, hence the claimed apparatus meets the criterion of comprising an inventive step.

### Embodiment of the surgery lamp with light emission control

The present invention will become easier to understand when considering the detailed description together with the appended drawings of the present invention, while the description should not be considered restrictive, but merely as an example to help explain and understand the invention.

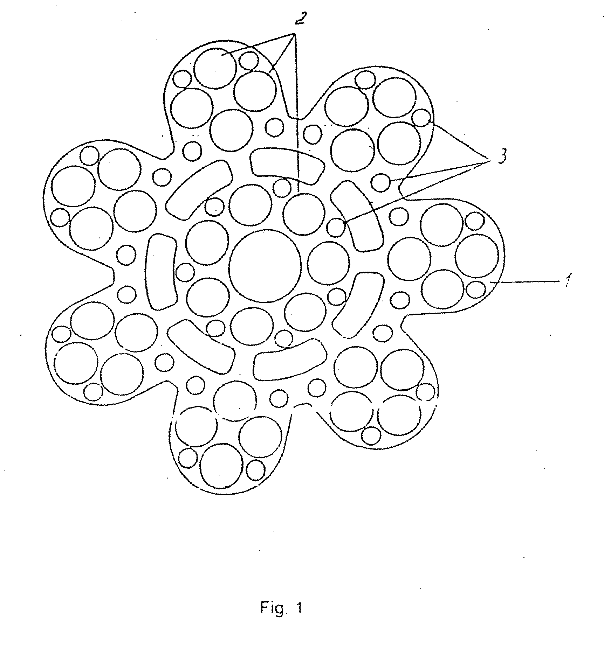

- Fig. 1: Schematic arrangement of the basic and additional light diodes on a surgery lamp.

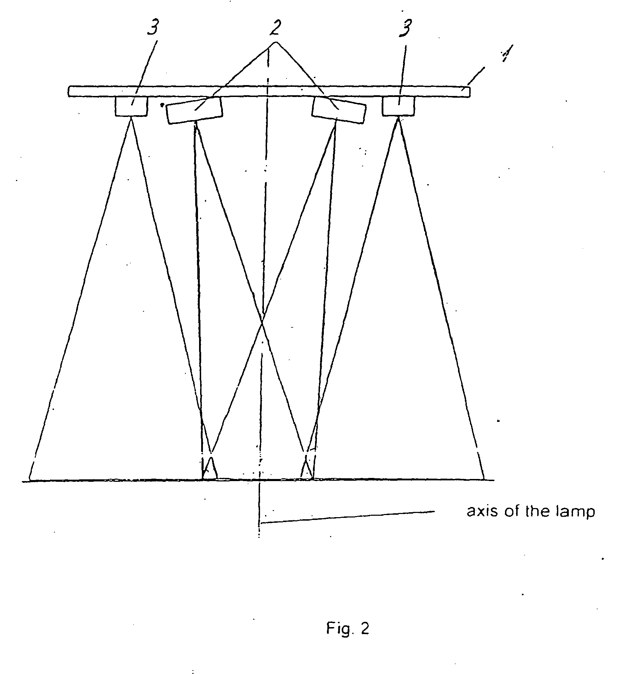

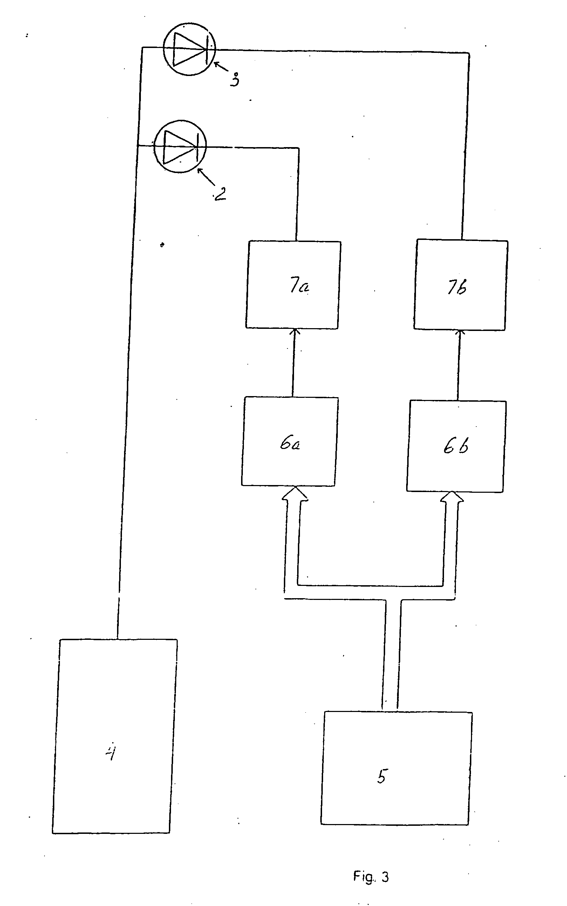

- Fig. 2: Path of the light rays in the lamp.
- Fig.: 3 Apparatus for controlling the light field of the surgery lamp.

The basic light diodes 2 and the additional light diodes 3, which are gathered in groups, are attached to the housing 1 of the lamp. The basic light diodes 2 have a width of the light beam equalling 6.5° and are focussed in such a way that their optical axes meet in the centre of the light spot at a distance of I m from the surface. The additional light diodes 3 have a width of the light beam equalling 10° and are distributed between the basic light diodes 2, their optical axes forming a concentric light spot in the shape of a ring around the basic light spot with a radius equalling 100 to 200 mm from the centre of the spot, which is optimal for surgery lamps. The apparatus for controlling the light field consists of a feed unit 4, a control unit with a microcomputer 5, digital-to-analogue converters 6a and 6b, electricity generators 7a and 7b.

When the lamp is connected to a supply grid, the supply from the feed unit 4 simultaneously goes to the groups of the basic light diodes 2 and the additional light diodes 3. When a large area requires illumination (in wide wounds), the groups of basic light diodes 2 and additional light diodes 3 are switched on. In deep wounds, when a light spot with small dimensions is required, only the groups of basic diodes 2 are switched on. In endoscopy, only an illumination of an area where the surgery takes place with the help of an endoscope is necessary, therefore only the groups of additional light diodes 3 are switched on. In these cases a correction of the balance of currents running through the groups of basic light diodes 2 and additional light diodes 3 is carried out with the help of the microcontroller with the control unit 5, the digital-to-analogue converters 6a and 6b and the generators 7a and 7b, which causes a redistribution of the light beam within the light field.

### Industrial applicability

The invention can be used in medical technology.

## Claims

1. Surgery lamp with light emission control, comprising
- light optical elements mounted on a lamp housing and having the form of basic light diodes (2) distributed over the surface of the lamp housing (1) as well as additional light diodes (3) arranged between the basic light diodes (2), and
- an apparatus for controlling the light field having a control unit (5) to which the groups of basic light diodes (2) and the groups of additional light diodes (3) are connected by regulators, each of which is connected to a feed unit (4),
wherein the basic and additional light diodes (2, 3) are gathered in groups,
wherein the groups of basic light diodes (2) and the groups of additional light diodes (3) are connected to each other and to the apparatus (4, 5, 6a, 6b, 7a, 7b) for controlling the light field,
**characterised in that**
the groups of basic and additional light diodes (2, 3) have different spatial orientation, and
the control unit (5) is used for correction of balance of currents through the groups of the basic light diodes (2) and the additional light diodes (3), causing thereby a redistribution of a light beam within the light field and allowing thereby to carry out an electronic control of the diameter of the light field.

2. Surgery lamp according to claim 1, **characterised in that** the basic light diodes (2) are mounted on the lamp housing (1) and their optical axes intersect the axis of the lamp in the centre of the light spot.

3. Surgery lamp according to claim 1, **characterised in that** the additional light diodes (3) are mounted on the housing (1) so that their optical axes form a concentric light spot in the shape of a ring around the basic spot.

## Patentansprüche

1. Chirurgielampe mit Lichtemissionssteuerung, umfassend
- lichtoptische Elemente, die an einem Lampengehäuse angebracht sind und die Form von Basislichtdioden (2), die über die Oberfläche des Lampengehäuses (1) verteilt sind, sowie von zusätzlichen Lichtdioden (3) aufweisen, die zwischen den Basislichtdioden (2) angeordnet sind, und
- eine Vorrichtung zur Steuerung des Lichtfelds, die eine Steuereinheit (5) aufweist, mit der die Gruppen von Basislichtdioden (2) und die Gruppen von zusätzlichen Lichtdioden (3) durch Reguliereinrichtungen verbunden sind, von denen jede mit einer Versorgungseinheit (4) verbunden ist,
- wobei die Basis- und die zusätzlichen Lichtdioden (2, 3) in Gruppen gesammelt sind,
- wobei die Gruppen von Basislichtdioden (2) und die Gruppen von zusätzlichen Lichtdioden (3) miteinander und mit der Vorrichtung (4, 5, 6a, 6b, 7a, 7b) zur Steuerung des Lichtfelds verbunden sind,
**dadurch gekennzeichnet, dass**
- die Gruppen von Basis- und zusätzlichen Lichtdioden (2, 3) eine unterschiedliche räumliche Ausrichtung aufweisen und
- die Steuereinheit (5) zur Korrektur des Gleichgewichts von Strömen durch die Gruppen der Basislichtdioden (2) und der zusätzlichen Lichtdioden (3) verwendet wird, wodurch eine Umverteilung eines Lichtstrahls innerhalb des Lichtfelds verursacht wird und wodurch die Durchführung einer elektronischen Steuerung des Durchmessers des Lichtfelds ermöglicht wird.

2. Chirurgielampe nach Anspruch 1, **dadurch gekennzeichnet, dass** die Basislichtdioden (2) an dem Lampengehäuse (1) angebracht sind und ihre optischen Achsen die Achse der Lampe im Zentrum des Lichtpunkts schneiden.

3. Chirurgielampe nach Anspruch 1, **dadurch gekennzeichnet, dass** die zusätzlichen Lichtdioden (3) an dem Gehäuse (1) so angebracht sind, dass ihre optischen Achsen einen konzentrischen Lichtpunkt in Form eines Rings um den Basispunkt herum bilden.

## Revendications

1. Lampe de chirurgie avec commande d'émission de lumière, comprenant
- des éléments optiques lumineux montés sur un boîtier de lampe et ayant la forme de diodes lumineuses de base (2) distribuées sur la surface du boîtier de lampe (1), ainsi que des diodes lumineuses additionnelles (3) agencées entre les diodes lumineuses de base (2), et
- un appareil pour commander le champ lumineux, ayant une unité de commande (5) à laquelle les groupes de diodes lumineuses de base (2) et les groupes de diodes lumineuses additionnelles (3) sont connectés par des régulateurs, chacun desquels est connecté à une unité d'alimentation (4),
dans laquelle les diodes lumineuses de base et additionnelles (2, 3) sont réunies en groupes,
dans laquelle les groupes de diodes lumineuses de base (2) et les groupes de diodes lumineuses additionnelles (3) sont connectés les uns aux autres et à l'appareil (4, 5, 6a, 6b, 7a, 7b) pour commander le champ lumineux,
**caractérisé en ce que**
les groupes de diodes lumineuses de base et additionnelles (2, 3) ont une orientation spatiale différente, et
l'unité de commande (5) est utilisée pour une correction d'équilibre de courants entre les groupes des diodes lumineuses de base (2) et des diodes lumineuses additionnelles (3), causant ainsi une redistribution d'un faisceau lumineux au sein du champ lumineux et permettant ainsi d'effectuer une commande électronique du diamètre du champ lumineux.

2. Lampe de chirurgie selon la revendication 1, **caractérisée en ce que** les diodes lumineuses de base (2) sont montées sur le boîtier de lampe (1) et leurs axes optiques coupent l'axe de la lampe au centre du point lumineux.

3. Lampe de chirurgie selon la revendication 1, **caractérisée en ce que** les diodes lumineuses additionnelles (3) sont montées sur le boîtier (1) de façon à ce que leurs axes optiques forment un point lumineux concentrique sous la forme d'un anneau autour du point de base.
